# EUROPÄISCHE PATENTANMELDUNG

(11) **EP 1 033 363 A1**
(43) Veröffentlichungstag der Anmeldung: **06.09.2000**
(21) Anmeldenummer: 99115402.2
(22) Anmeldetag: 04.08.1999
(51) Int. Cl.: C07C 233/18, A61K 7/08

(54) **Verfahren zur Herstellung von Verdickungsmitteln auf Basis von Fettsäure-monoisopropanolamid, ihre Verwendung und diese enhaltende Zubereitungen**

(30) Priorität: 02.03.1999 DE 19908944
(71) Anmelder: Goldschmidt Rewo GmbH & Co. KG, 36392 Steinau (DE)
(72) Erfinder: Hohn-Stöcker, Elke, 36381 Schlüchtern (DE); Möller, Christl, 36396 Steinau an der Strasse (DE); Bayle, Patrice, 27950 St. Marcel (FR)

(57) **Zusammenfassung**

Die Erfindung betrifft Verdickungsmittel auf Basis von Fettsäure-monoisopropanolamid, welche mit Ethylenoxid und/oder Propylenoxid verethert sind, seine Verwendung in tensidische Formulierungen und Formulierungen mit einem wirksamen Gehalt an diesen Verdickungsmitteln.

## Beschreibung

Die Erfindung betrifft Verdickungsmittel auf Basis von Fettsäure-monoisopropanolamid, welche mit Ethylenoxid und/oder Propylenoxid verethert sind, seine Verwendung in tensidische Formulierungen und Formulierungen mit einem wirksamen Gehalt an diesen Verdickungsmitteln.

Flüssige Detergentienmischungen für Haushalt und Industrie wie insbesondere Geschirrspülmittel, Waschmittel, Reinigungsmittel, Reinigungsgele für den Sanitärbereich; für kosmetische Produkte wie beispielsweise Haar-Shampoos, Duschgele und Flüssigseifen weisen in der Praxis häufig nicht die gewünschte viskose Konsistenz auf, was einmal aus anwendungstechnischem Aspekt nachteilig ist, insbesondere aber bei Produkten der persönlichen Anwendung in Körperreinigung, Körperpflege und Kosmetik als unvorteilhaft kritisiert wird.

Bei einer zu geringen Viskosität ist die ausreichend geringe Dosierung nicht ganz einfach, der Kontakt und die Verweilzeit/Einwirkungszeit auf den jeweiligen Oberflächen ist zu kurz und bei der manuellen Anwendung im persönlichen Bereich neigen die Produkte dazu, zwischen den Fingern zu verlaufen.

Es wurden daher in der Vergangenheit eine Vielzahl von Verdickungsmitteln für tensidische Zubereitungen vorgeschlagen, wie beispielsweise Fettsäureamide auf Basis von Dialkanolaminen wie Diethanolamin (US-A- 3,856,711). Diese sind jedoch aufgrund der potentiellen Nitrosaminbildung und des damit verbundenen cancerogenen Potentials nicht mehr allgemein akzeptabel. Verdickungsmittel auf Basis von Fettsäuren und Monoisopropanolamin weisen diesen Nachteil zwar nicht auf, jedoch tritt ihre verdickende Wirkung erst bei erhöhten Amidkonzentrationen auf, und sie erreichen ihre maximale /optimale Verdickungswirkung nur bei Mitverwendung genügend hoher Mineralsalzmengen. Grosse Mengen an Mineralsalzen sind aus anwendungstechnischer Sicht nicht erwünscht, da sie Korrosionsprobleme bei der Lagerung bereiten können und bei niedrigen Temperaturen weniger stabile Formulierungen ergeben. Üblicherweise sind die Monoisopropanolamide der allgemein verwendeten Fettsäuren pastös oder fest, dies stellt ebenfalls ein Hindernis bei der Anwendung dar, da ein erhöhter Energiebedarf zur Einarbeitung in Formulierungen.erforderlich ist.

In der EP-B-0 574 277 wird die Verwendung des Monoisopropanolamids der Isostearinsäure als Verdickungsmittel vorgeschlagen. Dieses weist eine Verbesserung der verdickenden Eigenschaften bereits in geringen Mengen und bei verminderten Mineralsalzkonzentrationen auf. Das Produkt ist ausserdem flüssig und relativ leicht bei Raumtemperatur (RT) zu verarbeiten.

Aus anwendungstechnischer Sicht ist damit ein wesentlicher Fortschritt erzielt worden, jedoch sind diese Verbindungen hinsichtlich ihrer Lagerstabilität, insbesondere bei niedrigen Temperaturen, noch verbesserungsbedürftig.

Es hat sich nämlich gezeigt, dass im Laufe der Lagerung feste Anteile aus dem anfangs klaren Produkt sedimentieren und sich als fester Bodenbelag absetzen. Vor der Anwendung müssen diese daher erst aufgeschmolzen und durch intensives Rühren wieder homogenisiert werden. Die Verwendung sowohl des dekantierten klaren als auch des festen Anteils allein erreichen in ihren Einzelwirkungen nicht die Gesamtwirkung.

Daneben bedeutet diese Vorgehensweise natürlich einen unerwünschten Ausbeuteverlust und/oder zusätzliche technische Aufwände für den Formulierer. Darüberhinaus ist die Gefahr nicht auszuschliessen, dass Lager- und/oder andere Probleme auch in den Endformulierungen auftreten können.

Eine Aufgabe der vorliegenden Erfindung war es daher, diese Probleme des bekannten Produktes zu vermeiden und seine Lagerstabilität sowohl bei Raumtemperatur als auch bei niedrigen Temperaturen zu verbessern.

Überraschenderweise wurde nun gefunden, dass durch Umsetzung des Isostearinsäure-monoisopropanolamids sowie des Canolafettsäure-monoisopropanolamids mit Propylenoxid und/oder Ethylenoxid diese Aufgabe gelöst werden kann.

Ein Gegenstand der Erfindung sind daher Verbindungen der allgemeinen Formel (I)

CₙH₂ₙ₊₁-C(O)NH - CH₂ - CH(CH₃)- O-(CH₂-CH(R)-O)ₘ-H

in welcher n = 13 bis 17, insbesondere ein Isostearoylrest oder ein Canolafettsäurerest, R = H, CH₃ und m einen Wert von 1,5 bis 5,5 insbesondere 2,5 bis 4,0 annehmen kann.

Die Amidbildung der ersten Stufe kann nach den bekannten Herstellungsverfahren von Amiden erfolgen. Vorzugsweise wird das in der EP-B-0 574 277 beschriebene Verfahren benutzt, bei dem man eine stöchiometrische Menge oder einen leichten Überschuss (ca. 1,1 mol) an Isopropanolamin (auch 1-Amino-2-propanol, 2-Hydroxypropylamin, Monoisopropanolamin oder kurz MIPA genannt) zu der auf 40-70 °C vorgewärmten und mit Antioxidans vorbehandelten Isostearinsäure oder der Canolafettsäure zugibt und die Temperatur auf 90 bis 110 °C steigen lässt. Danach werden als Katalysator 2-5 Gew.-Teile Phosphorsäure/ 10.000 Gew.-Teile Isostearinsäure oder der Canolafettsäure eingetragen und die Temperatur auf 145-170 °C erhöht. Sobald die Säurezahl der Mischung kleiner als 5 (mg KOH/g) ist, wird eine zweite Menge Antioxidans hinzugegeben und bei vermindertem Druck von 4-1,9x10³ Pa die Reaktion vervollständigt und der verwendete Überschuss an MIPA entfernt.

Die anschliessende Ethoxylierung und/oder Propoxilierung wird bei ca 150-170 °C im alkalischen Medium durchgeführt.

Der mittlere Propoxylierungs- bzw. Ethoxlierungsgrad (n) dieser Homologengemische entspricht dem Verhältnis der Stoffmengen Ethylenoxid/Propylenoxid und Isostearinsäuremonoisopropanolamid oder Canolafettsäuremonoiso-propanolamids, mit denen die Anlagerungsreaktion durchgeführt wird.

Die erfindungsgemässen Verdickungsmittel sind mit fast allen in der Kosmetik, Haar- und Körperreinigung und im Haushalt und in der Industrie für Reinigungszwecke und im Sanitärbereich üblicherweise verwendeten oberflächenaktiven Verbindungen verträglich. Die Verbindungen können einzeln oder als Mischungen verwendet werden und sind beispielsweise mit anionischen, nichtionischen und amphoteren Tensiden wie Alkali-, Ammonium- oder Magnesium-Alkylsulfaten bzw. Alkylethersulfaten, sekundäre Alkansulfonate, Alkali-a-Olefinsulfonate, Sulfosuccinaten, Acylisethionaten, Sarkosiden, Tauriden, Alkylpolyglukosiden, Ethercitraten, Carboxylaten, Ethercarboxylaten, Alkylamidethersulfaten, sowie Ethoxilaten von Fettalkoholen, Glyceriden, Ölen, Fettsäuren aber auch Fettsäureestern, Aminoxiden, Alkylbetainen, Alkylamidobetainen, Propionaten, Glyinaten, Acetaten und Sulfobetainen und Natrium-, Kalium-oder Triethanolaminseifen verträglich.

Die erfindungsgemässen viskositätssteigernden Mittel werden im allgemeinen in Mengen von 0,1 bis ca 5 Gew.-%, vorzugsweise zwischen 0,5 bis 3,0 Gew.-%, bezogen auf Gesamtformulierung, mitverwendet. Ihr wirksamer Anteil ist abhängig von der Art und der Menge der jeweiligen Tenside oder deren Mischungen und kann durch wenige einfache orientierende Versuche in Kombination mit den mitverwendeten Mineralsalzen optimiert werden.

Als Mineralsalze werden insbesondere die Chloride oder Sulfate der Alkali- oder Erdalkalimetalle in Mengen von etwa 0,1 bis 10 Gew.-%, bezogen auf die Gesamtformulierung, mitverwendet.

Die nachstehend gegebenen Beispiele werden mit einer handelsüblichen Isostearinsäure durchgeführt, welche gemäss gaschromatisch ermittelter Analysenwerte die folgende mittlere Kettenlängenverteilung aufweisen:

| | |
|---|---|
| C16 | 12,5% |
| C18 iso | 70,6 % |
| C18 | 4,4 % |
| C18' | 3,4 % |
| C18 | 9,4 % |

Die mitverwendete Canolafettsäure ist eine handelsübliche Fettsäure, welche welche gemäss gaschromatisch ermittelter Analysenwerte die folgende mittlere Kettenlängenverteilung aufweisen:

| | |
|---|---|
| C14 | 1,5 % |
| C 16 | 12,5% |
| C 18 | 4,5% |
| C18' | 72,0 % |
| C 18" | 9,5 % |

Das mitverwendete Monoisopropanolamin ist ein handelsübliches Produkt mit einer Reinheit von 99 Gew.-%.

### Beispiele

Die Herstellung des Monoisopropanolamids erfolgt gemäss Beispiel 1 der EP-B- 0 574 277 aus

| | |
|---|---|
| Isostearinsäure | 668 g |
| Monoisopropanolamin | 185 g |
| Phosphorsäure (85 %ig) | 0,24 g |
| 2,6-Di-tert-butyl-p-kresol | 1,92 g |
| (Antioxidans) | |

nach den dortigen Angaben.

| Analysendaten: | |
|---|---|
| Aussehen bei 20 °C | klare Flüssigkeit, neigt bei Lagerung zur Eintrübung |
| Farbe Gardner | 5 |
| Säurezahl mg KOH/g | 0,5 |
| % fr. Amin | 0,6 |
| pH Wert, 1 %ig (Wasser/Isopropanol, 1:1) | 8,6 |

Die Umsetzung mit Ethylenoxid bzw. Propylenoxid wurde im direkten Anschluss vorgenommen, die Anlagerung erfolgt bei 150-170 °C im alkalischen Medium nach den bekannten Verfahren.

### Beispiel 1

ca 3,5 EO Anlagerung

366 g des Reaktionsprodukts Beispiel 1 aus EP-B 0 574 277
154 g Ethylenoxid

| Analysendaten : | |
|---|---|
| Aussehen bei 20 °C | klare Flüssigkeit |
| Farbe Gardner | 2 |
| Esterzahl mg KOH/g | 18,7 |
| Hydroxylzahl mg KOH/g | 126,9 |

### Beispiel 2

Anlagerung 2,9 EO
366 g des Reaktionsproduktes Beispiel 1 aus EP-B 0 574 277
128 g Ethylenoxid

| Analysendaten: | |
|---|---|
| Aussehen bei 20 °C | klare Flüssigkeit |
| Farbe Gardner | 2,3 |
| Esterzahl mg KOH/g | 20,1 |
| Hydroxylzahl mg KOH/g | 137,1 |

### Beispiel 3

Anlagerung von 5 EO

366 g des Reaktionsproduktes aus Beispiel 1 der EP-B 0 574 277 220 g Ethylenoxid

| Analysendaten: | |
|---|---|
| Aussehen bei 20 °C | klare Flüssigkeit |
| Farbe Gardner | 2 |
| Esterzahl mg KOH/g | 19,1 |
| Hydroxylzahl mg KOH/g | 119 |

### Beispiel 4

Anlagerung von 1,5 PO

366 g des Reaktionsproduktes aus Beispiel 1 der EP-B 0 574 277 87,1 g Propylenoxid

| Analysendaten: | |
|---|---|
| Aussehen bei 20 °C | klare Flüssigkeit |
| Farbe Gardner | 3 |

### Beispiel 5

Anlagerung von 5 PO

366 g des Reaktionsproduktes aus Beispiel 1 der EP-B 0 574 277
290 g Propylenoxid

| Analysendaten: | |
|---|---|
| Farbe Gardner | 2,6 |
| Esterzahl mg KOH/g | 27,7 |
| Hydroxylzahl mg KOH/g | 122 |

### Beispiel 6

311 g Canolafettsäure
90 g MIPA
132 g Ethylenoxid

| Analysendaten: | |
|---|---|
| Farbe Gardner | 2,8 |
| Säurezahl | 4,1 |

### Anwendungstechnische Ergebnisse

### Lagerstabilität

Bei vorzugsweise normalen (Raumtemperatur von ca 20 °C) und niedrigen Temperaturen (< 10 °C) von Produkten mit variierendem EO/PO-Gehalt liegt die Lagerstabilität bei ca. 4-6 Monaten. Bei höheren Temperaturen von ca. 50-60 °C liegt die Lagerstabilität bei ca. 1 Monat.

### Verdickungswirkung

Beispielsrezepturen in Anlehnung an EP-B- 0 574 277.

| Tensid Mischung Viskositätstest | Nr. 1 | Nr.2 | Nr. 3 |
|---|---|---|---|
| Sodium Laureth Sulfate (NLES) | 10 % * | 10 % * | 10 %* |
| Isostearins.MIPA | 2 % | - | - |
| Isostearins.MIPA + 3,5 EO | - | 2 % | - |
| Isostearins. MIPA + 2,9 EO | - | - | 2 % |
| Natriumchlorid | 1 - 5 % | 1-5 % | 1 -5 % |
| Wasser | ad 100 | ad 100 | ad 100 |
| Citronensäure zur pH Wert Einstellung auf ca 6,0 | | | |

| | | | |
|---|---|---|---|
| *(jeweils auf Feststoff ber.) | | | |

### Verdickungswirkung:

Beispielrezepturen in Anlehnung an die Tensidmischung der EP-B 0 574 277

| Viskositätstest | Nr. 1 | Nr.2 | Nr. 3 |
|---|---|---|---|
| Sodium Laureth Sulfate (NLES) | 10 % * | 10 % * | 10 %∗ |
| Canolafetts.MIPA | 2 % | - | - |
| Canolafetts.MIPA + 3,0 EO | - | 2 % | - |
| Natriumchlorid | 1 - 5 % | 1-5 % | 1 -5 % |
| Wasser | ad 100 | ad 100 | ad 100 |

Citronensäure zur pH Wert Einstellung auf ca 6,0

Viskositäten der Testreihe nach Brookfield in mPa*s, bei 20 °C:

| % NaCl- Gehalt | 1,0 | 1,5 | 2,0 | 2,5 | 3,0 | 4,0 | 4,5 |
|---|---|---|---|---|---|---|---|
| Rez Nr 1 | 92 | 1500 | 14400 | 28100 | 28100 | 7580 | 1750 |
| Rez. Nr 2 | 28 | 44 | 432 | 3970 | 14400 | 31300 | 39200 |
| Rez.Nr 3/NLES | - | - | 28 | - | 660 | 12500 | 28300 |

### Ide Flüssigseifen

| mit unterschiedlichen Co-Tensiden | Nr. 1 | Nr. 2 |
|---|---|---|
| Natriumlaurylethersulfat | 10 % * | 10 % * |
| Disodium Laureth Sulfosuccinate | 2 %* | - |
| Sodium Cocoamphoacetate | - | 2 % * |
| Isostearins. MIPA**+ 3 EO | 1,5 %* | 1,5 % * |
| Wasser | ad 100 | ad 100 |
| Citronensäure zur pH Wert Einstellung auf ca 5,5 | | |

| | | |
|---|---|---|
| *(jeweils auf Feststoff ber.); | | |
| ** Monoisopropanolamid | | |

| Viskositäten bei 20°C, nach Brookfield | | Rez. Nr.1 | Nr. 2 |
|---|---|---|---|
| % NaCl Zusatz | 1,5 | 28 mPas | 2000 mPas |
| | 1,7 | 40 mPas | 4900 mPas |
| | 3,2 | 1820 mPas | |
| | 3,5 | 6350 mPas | |

### Flüssigseife mit konditionierendem Zusatz, hautglättend

| | |
|---|---|
| Cocamidopropyl Betain, 45 % | 10 % |
| NLES 2 EO, 28 % | 35 % |
| Isostearins. MIPA+ 3 EO | 2,0 % |
| Ricinoleamidopropyltrimonium Methosulfate | 1,5 % |
| Wasser | ad 100 % |

Citronensäure zur pH-Wert Einstellung auf ca. 5,5
Konservierungsmittel, Farbe, Parfüm n.B.
Viskosität bei 20 °C, nach Brookfield ca 3000 mPas mit ca. 1,5 % NaCl Schaumkraft nach Ross Miles 140/135 mm

### Extrem mildes Baby Shamooo, frei von NLES

| | |
|---|---|
| Cocamidopropyl Betain, 45 % | 12 % |
| Disodium Laureth Sulfosuccinate, 40 % | 20 % |
| Sodium Cocoamphoacetate, 40% | 5 % |
| Isostearins. MIPA+ 3 EO | 2 % |
| PEG-200 Hydrogenated Glyceryl Palmate | 4 % |
| Wasser | ad 100 % |

Citronensäure zur pH-Wert Einstellung auf 5,5

Viskosität bei 20 °C, nach Brookfield: ca. 5000 mPas
Schaumkraft nach Ross Miles: 130/120 mm

## Patentansprüche

1. Verbindungen der allgemeinen Formel (I)
CₙH₂ₙ₊₁-C(O)NH - CH₂ - CH(CH₃) - O-(CH₂-CH(R)-O)ₘ-H
in welcher n = 13 bis 17, R = H, CH₃ und m einen Wert von 1,5 bis 5,5 annehmen kann.

2. Verbindungen der Formel 1, dadurch gekennzeichnet, dass m einen Wert von 2,5 bis 5,0 aufweist.

3. Verbindungen der Formel 1, dadurch gekennzeichnet, dass die Fettsäure Isostearinsäure oder Canolafettsäure ist.

4. Verwendung einer wirksamen Menge einer Verbindung gemäss Anspruch 1 als Verdickungsmittel in einer mindestens ein oberflächenaktives Mittel enthaltenden Zubereitung.

5. Zubereitungen, in denen mindestens 80 Gew.-% des als Verdickungsmittel in tensidhaltigen Zubereitungen verwendeten Verdickers aus Verbindungen der Formel (I)
CₙH₂ₙ₊₁-C(O)NH - CH₂ - CH(CH₃) - O-(CH₂-CH(R)-O)ₘ-H
in welcher n = 13 bis 17, R = H, CH₃ und m einen Wert von 1,5 bis 5,5 annehmen kann.

6. Flüssige oder pastöse Zubereitungen, enthaltend
0,5-4,0 Gew.-Teile einer Verbindung gemäss den Ansprüchen 1 bis 3
1,0-5,0 Gew.-Teile Mineralsalz
10-25 Gew.-Teile mindetens eines Tensides
ad 100 Gew.-Teile Wasser und gegebenenfalls Konservierungsmittel, Parfümöle, Farbstoffe, übliche Hilfs- und Zusatzstoffe.
